(19) Europäisches Patentamt
European Patent Office
Office européen des brevets

(11) Publication number: **0 033 629**
**B1**

(12) # EUROPEAN PATENT SPECIFICATION

(45) Date of publication of patent specification: **05.12.84**

(51) Int. Cl.$^3$: **C 07 C 79/46**, C 07 C 103/34, A 01 N 37/48, C 07 C 103/16

(21) Application number: **81300349.8**

(22) Date of filing: **27.01.81**

(54) **2-Nitro-5-(substituted-phenoxy) benzoate esters of hydroxyalkanoic acids and derivatives thereof as herbicides.**

(30) Priority: **01.02.80 US 117753**

(43) Date of publication of application: **12.08.81 Bulletin 81/32**

(45) Publication of the grant of the patent: **05.12.84 Bulletin 84/49**

(84) Designated Contracting States: **AT BE CH DE FR IT LI NL SE**

(56) References cited: **EP-A-0 020 052**

(73) Proprietor: **RHONE-POULENC AGROCHIMIE**
**14-20, rue Pierre Baizet**
**F-69009 Lyon (FR)**

(72) Inventor: **Theissen, Robert James**
**474 Van Holten Road**
**Bridgewater, New Jersey 08807 (US)**

(74) Representative: **Brachotte, Charles et al**
**RHONE-POULENC AGROCHIMIE 14-20, Rue Pierre Baizet B.P. 9163**
**F-69263 Lyon Cedex 1 (FR)**

**EP 0 033 629 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention is concerned with certain herbicidal 2-nitro-5-(substituted-phenoxy) benzoate esters of hydroxy-alkanoic acids and derivatives thereof.

Applicant Theissen herein has been granted a series of patents relating to 2-nitro-5-(substituted-phenoxy) benzoic acid derivatives including the salt, alkyl and cycloalkyl esters U.S. Patent No. 3 652 645; 3 784 635; 3 873 302; 3 983 168 and 3 907 866 describe certain phenoxy benzoic acid derivatives which are useful as herbicides.

Also European patent application 20052 describes some other phenoxy benzoic acid derivatives. However this application teaches that, in this area, herbicidal effectiveness cannot be predicted and often quite closely related compounds will have quite different weed control abilities.

We have now discovered further phenoxy benzoic acid derivatives which have good activity as herbicides.

This invention relates to certain herbicidal compounds within the class of 2-nitro-5-(substituted-phenoxy) benzoate esters of hydroxy alkanoic acids and derivatives thereof wherein there are up to six carbon atoms in the alkanoic acid moiety. Preferred members of this class are the $\alpha$-hydroxy alkanoic acids and derivatives which fall within the general formula:

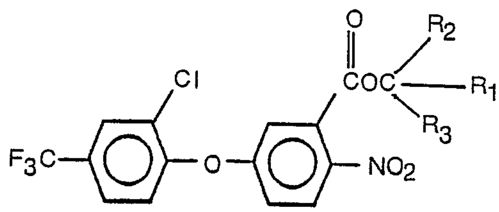

(I)

wherein

$R_1$ is selected from COOH; COOM where M is a cation selected from alkali metals, alkaline earth metals, ammonium, $alkyl_{1-5}$ and $dialkyl_{2-6}$ ammonium; COO $R_7$ where $R_7$ is $alkyl_{1-5}$; and

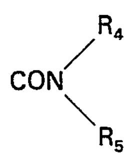

where $R_4$ and $R_5$ are selected from hydrogen and $alkyl_{1-5}$;

$R_2$ and $R_3$ may be the same or different and each is selected from hydrogen; $alkyl_{1-5}$; $QCOOR_6$ where Q is $alkylene_{0-3}$, $R_6$ is $alkyl_{1-5}$, hydrogen, a cation selected from alkali metals, alkaline earth metals, ammonium, $alkyl_{1-5}$ and $dialkyl_{2-6}$ ammonium; and

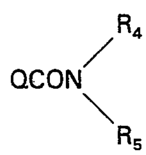

at least one of $R_2$ and $R_3$ being selected from

$QCOOR_6$ and 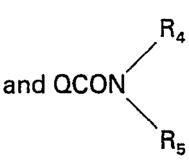

The term "alkyl" means straight or branched carbon chains where the carbon length is in the range set forth in the adjacent subscript.

One method for preparing these compounds is the use of the Ullman ether synthesis reaction between the alkali metal (e.g., Na, K) salt of a suitable substitued phenol, e.g., m-hydroxy benzoic acid or m-cresol with an active halogen-substituted aromatic, e.g., 3,4-dichlorobenzotrifluoride. The intermediate obtained may be nitrated and subsequently derivatized. Where m-cresol is used as a starting material, the product obtained can be oxidized and subsequently nitrated before the derivatization. The derivatization steps may be carried out by known techniques. One such technique is the reaction of the appropriate halo-alkanoic acid or derivative with the salt of the particular 2-nitro-5(substituted phenoxy) benzoic acid. Another technique is the reaction of the desired hydroxy alkanoic acid or derivative with the particular 2-nitro-5(substituted phenoxy) benzoyl halide.

Illustrative $\alpha$-hydroxy alkanoic acids and derivatives of the present invention are listed in Table I.

TABLE I

| $R_1$ | $R_2$ | $R_3$ |
|---|---|---|
| $COOC_2H_5$ | H | $COOC_2H_5$ |
| COOH | $CH_2COOH$ | $CH_2COOH$ |
| $COOCH_3$ | H | $(CH_2)_3COOCH_3$ |
| COONa | H | $CH_2COONa$ |
| $CONH_2$ | H | $CONH_2$ |
| COOH | H | COOH |
| COOH | COOH | $CH_3$ |

The compounds of this invention can be applied in various ways to achieve herbicidal action. They can be applied per se, as solids or in vaporized form, but are preferably applied as the toxic components in pesticidal compositions of the compound and a carrier. These compositions are preferably applied directly to the soil and often incorporated therewith. The compositions can be applied as granulars or dusts; as liquid sprays, or as gas-propelled sprays and can contain, in addition to a carrier, additives such as emulsifying agents, binding agents, gases compressed to the liquid state, odorants, stabilizers, and the like. A wide variety of liquid and solid carriers can be used. Non-limiting examples of solid carriers include talc, bentonite, diatomaceous earth, pyrophyllite, fullers earth, gypsum, flours derived from cotton seeds and nut shells, and various natural and synthetic clays having a pH not exceeding about 9.5. Non-limiting examples of liquid carriers include water, organic solvents such as alcohols, ketones, light oils, and medium oils and vegetable oils such as cottonseed oil.

In practice, herbicidal application is measured in terms of pounds of herbicide applied per acre. The compounds of this invention are effective herbicides when applied in herbicidal amounts, e.g., at rates between about 0.2 and 10 kg. per hectare.

The pesticidal compositions comprising the active ingredient and the carrier may be supplied either as ready-to-use compositions or as concentrates. Concentrates generally contain a higher proportion of the active ingredient than the ready-to-use compositions and accordingly, require dilution prior to use. Both the ready-to-use compositions and the concentrates may be in liquid or solid form, i.e., with the active ingredient blended with a liquid or solid carrier, respectively.

The amount of active ingredient in the composition will depend, primarily, upon whether the composition is a concentrate or a ready-to-use composition. Concentrates will generally contain from 5 to 95% by weight, preferably 10 to 80% by weight, of the active ingredient. The concentration of active ingredient in the ready-to-use compositions will vary according to the method of application for the composition in question and to the desired application rate. In general, ready-to-use compositions will contain from 0.001 to 1, preferably 0.01 to 0.1 percent by weight of the active ingredient. Thus, the compositions may contain from 0.001 to 95% by weight of active ingredient, the actual amount depending upon the nature of the composition and the method by which it shall be applied.

Concentrates may be either liquid or solid and are usually extendable with water to form emulsions of suspensions containing a smaller proportion of the active ingredient. Alternatively, liquid or solid concentrates may be extended with liquid or solid carriers to give the final ready-to-use composition. Liquid concentrates preferably comprise the active ingredient and an emulsifying agent dissolved in a liquid solvent, e.g., an organic solvent of the type mentioned above. The emulsifier is suitably an anionic, cationic or non-ionic emulsifier, e.g., sodium dodecylbenzensulfonate or an ethylene oxide derivative of an alkyl pheonol, a mercaptan, an amine or a carboxylic acid. Liquid concentrates may advantageously contain from 10 to 30 weight percent, e.g., 25 weight percent of the active ingredient, e.g., 1 kg. of active ingredient per 4 kg. of concentrate.

Wettable powders are another preferred form of concentrate and these suitably comprise the active ingredient, a finely-divided solid carrier and at least one surfactant to impart wettability or dispersability. Solid carriers which are suitable for preparing wettable powder formulations may be either organic or inorganic in nature. Suitable organic carriers are soybean, walnut, or wood flour or tobacco dust, and suitable inorganic ones are clays of the bentonite, kaolinite, or fuller's earth types; silicas such as diatomaceous earth; silicates such as talc, pyrophyllite, or alkaline earth silicates; and

calcium and magnesium carbonates. The carrier may be a single substance or a mixture of finely divided solids. A surfactant or mixture of surfactants is generally present in an amount of 1 to 10 percent by weight of the wettable powder formulation. Suitable dispersing agent are sodium formaldehyde-naphthalene sulfonate or sodium lignin sulfonate. Wetting agents include higher alkylaryl sulfonates ("higher alkyl" meaning alkyl of at least 8 carbon atoms) such as calcium dodecylbenzenesulfonate, alcohol sulfates, alkylphenoxyethoxyethoxyethyl sodium sulfonates, sodium dioctyl sulfosuccinate, and ethylene oxide adducts with fatty alcohols, fatty acids, or with higher alkyl-phenols, such as octylphenoxypolyethoxyethanol. Sticking or spreading agents may be included such as glycerol mannitan laurate or a condensate of polyglycerol and oleic acid modified with phthalic anhydride. The active ingredient content of the wettable powder may be in the range of 20 to 80% by weight; however, the preferred range of concentrations is 50% to 80%.

Dusts may be made by incorporating the active ingredient into a solid carrier, such as finely powdered clays, talc, silica, and synthetic silicates, alkaline earth carbonates, and diluents of natural origin such as tobacco dust or walnut shell flour. Granular formulations can be made from similar type solid carriers except that the particle size is larger in the range of 15 to 60 mesh (U.S. Standard Sieve Series). A small amount of dispersing agent may be incorporated in these solid formulations. The concentration of active ingredients in these dust or granular formulations may be in the range of 1 to 20% by weight. The solid carriers used in these formulations may be essentially inert or they may consist wholly or in part of fertilizing materials such as ammonium sulfate, or other ammonium salts, urea, calcium phosphates, potassium chloride or dried blood.

One particularly convenient method for making solid formulations is to treat the solid carrier with the active ingredients dissolved in a solvent and allow the solvent to evaporate off. This results in the carrier which is usually in the form of finely divided particles, being impregnated with the active ingredients. Another method is to apply the mixture of active ingredients in the molten state or by spraying.

The concentration of the active ingredient in the final ready-to-use composition will depend not only upn the application rate desired (generally in the range of 0.2 to 10 kg. per hectare, as mentioned above), but also upon the application method which is to be used. Wettable powders and liquid concentrates are usually applied as aqueous sprays and applied at application rates varying from about 10 to 1500 liters per hectare. With ground equipment, the application rate will generally be in the range of 100 to 500 liters per hectare, whereas aerial spray equipment will generally apply 15 to 80 liters per hectare.

HERBICIDAL EFFECTIVENESS

*Method of Propagating Test Species*

Crop and week species are planted in 8″ × 10″ disposable fiber flats containing potting soil to provide each flat with a 4″ rot of all test species. Crop species consist of field corn (CN), crabgrass (CG), cotton (CT), and soybeans (SB). The weed species consists of foxtail millet (FM), green foxtail (GF), velvetleaf (VL), cocklebur (CB), wild mustard (WM) and pigweed (PW).

Cotton, corn, soybean, and cocklebur plantings consist of 4 to 5 seeds per row depending upon species. The smaller seeded species (velvetleaf, wild mustard, pigweed, foxtail millet and green foxtail) are planted in an uncounted but sufficient number to provide a solid row of seedlings.

Plantings for the pre and post-emergence portions of the test are identical as to seeding. The initial watering until emergence is done from the top. The post-emergence phase is propagated in advance so as to provide plants of the proper stage of development at the time of treatment. Plantings for the pre-emergence phase are made not more than one day in advance of treatment.

The desired stage of development for treatment of the post-emergence broadleaf species (CT, SB, CB, VL, WM, PW) is the one true leaf or first trifoliate leaf stage. The desired stage for corn would be a height of 3—4″, while a 2″ height would be adequate for the grasses.

*Method of Treatment*

Spray applications are made with a handgun sprayer (aspirator type) simultaneously to one flat of established plants for the post-emergence phase and one newly seeded flat for the pre-emergence phase. The 10 lb./acre treatment rate consists of the uniform application of 116 milligrams of test compound to the combined area of the two flats (160 sq. inches). Application is made in a solvent mixture consisting of 40 ml acetone and 40 ml water and a surfactant concentration of 0.1%.

Following spray application, flats are returned to the greenhouse where watering of the post-emergence phase is done only by subirrigation. The pre-emergence phase is top watered by sprinkling until after test species have emerged. Subsequent watering is by sub-irrigation.

Two weeks after treatment, the pre- and post-emergence injury and control is rated on a 0—100% injury and control scale. Special physiological effects are rates as to intensity also at this time.

**Claims**

1. Herbicidal compounds of the formula

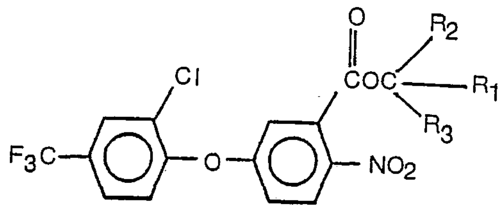

wherein

$R_1$ is selected from COOH; COOM where M is a cation selected from alkali metals, alkaline earth metals, ammonium, $alkyl_{1-5}$ and $dialkyl_{2-6}$ ammonium; COO $R_7$ where $R_7$ is $alkyl_{1-5}$; and

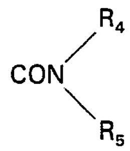

where $R_4$ and $R_5$ are selected from hydrogen and $alkyl_{1-5}$;

$R_2$ and $R_3$ may be the same or different and each is selected from hydrogen; $alkyl_{1-5}$; $QCOOR_6$ where Q is $alkylene_{0-3}$, $R_6$ is $alkyl_{1-5}$, hydrogen, a cation selected from alkali metals, alkaline earth metals, ammonium, $alkyl_{1-5}$ and $dialkyl_{2-6}$ ammonium; and

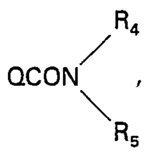 ,

at least one of $R_2$ and $R_3$ being selected from

$QCOOR_6$ and 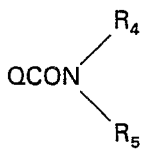

2. The compounds of Claim 1 wherein $R_2$ is hydrogen.
3. The compounds of Claim 2 wherein $R_3$ is $QCOOR_6$.
4. The compounds of Claim 3 wherein $R_1$ is $COOR_7$.
5. The compounds of Claim 3 wherein Q is $alkylene_{1-3}$.
6. The compounds of Claim 3 wherein $R_6$ is selected from the group of cations mentioned in Claim 1.
7. The compounds of Claim 3 wherein $R_6$ is hydrogen.
8. The compounds of Claim 3 wherein $R_6$ is $alkyl_{1-5}$.
9. The compounds of Claim 3 wherein $R_3$ is $COOR_6$.
10. The compounds of Claim 3 wherein $R_1$ is COOH.
11. The compounds of Claim 1 wherein $R_2$ is $alkyl_{1-5}$.
12. The compounds of Claim 2 wherein $R_3$ is

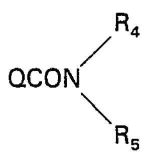

13. The compounds of Claim 1 wherein $R_2$ and $R_3$ are $QCOOR_6$.
14. The compounds of Claim 13 wherein $R_1$ is $COOR_7$.

15. A herbicidal composition comprising an herbicidally effective amount of a compound of Claim 1 and a carrier therefor.

16. A method for combating undesirable herbs, said method comprising contacting them with an herbicidally effective amount of a compound defined in Claim 1.

17. A method of preparing a compound as defined in Claim 1, which comprises reacting a compound of the formula

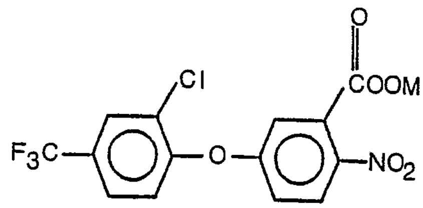

where M is as defined in Claim 1, with an alkanoic acid or halide thereof of the formula

$$XCR_1R_2R_3$$

where $R_1$, $R_2$ and $R_3$ are as defined in claim 1 and X is halogen.

18. A method of preparing a compound as defined in Claim 1, which comprises reacting a compound of the formula

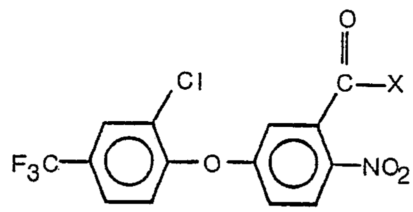

where X is halogen, with an alkanoic acid of the formula

$$HOCR_1R_2R_3$$

where $R_1$, $R_2$ and $R_3$ are as defined in Claim 1.

**Revendications**

1. Compositions herbicides de formule:

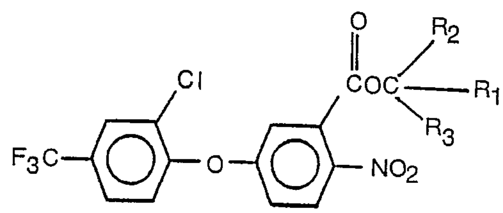

dans laquelle:

$R_1$ est choisi parmi COOH; COOM, M étant un cation choisi parmi les métaux alcalins, les métaux alcalino-terreux, l'ammonium, les alkyl 1—5 et dialkyl 2—6 ammonium; $COOR_7$ $R_7$ étant un alkyle 1—5; et $—CONR_4R_5$, $R_4$ et $R_5$ étant choisis parmi l'hydrogène ou un alkyle 1—5;

$R_2$ et $R_3$ peuvent être identiques ou différents et chacun est choisi parmi l'hydrogène; alkyle 1—5; Q $COOR_6$, Q étant alkylène 0—3 et $R_6$ étant alkyle 1—5, hydrogène, un cation choisi parmi les métaux alcalins, les métaux alcalino-terreux, l'ammonium, alkyle 1—5 et dialkyl 2—6 ammonium; et Q $CONR_4R_5$, au moins l'un de $R_2$ et $R_3$ étant choisi parmi

Q $COOR_6$ et Q $CONR_4R_5$.

2. Composés selon la revendication 1 dans lesquels $R_2$ est l'hydrogène.

3. Composés selon la revendication 2 dans lesquels $R_3$ est Q $COOR_6$.

4. Composés selon la revendication 3 dans lesquels $R_1$ est $COOR_7$.

5. Composés selon la revendication 3 dans lesquels Q est alkylène 1—3.

6. Composés selon la revendication 3 dans lesquels $R_6$ est choisi parmi le groupe de cations mentionnés dans la revendication 1.

7. Composés selon la revendication 3 dans lesquels $R_6$ est l'hydrogène.

8. Composés selon la revendication 3 dans lesquels $R_6$ est alkyle 1—5.

9. Composés selon la revendication 3 dans lesquels $R_3$ est $COOR_6$.

10. Composés selon la revendication 3 dans lesquels $R_1$ est COOH.

11. Composés selon la revendication 1 dans lesquels $R_2$ est alkyle 1—5.

12. Composés selon la revendication 2 dans lesquels $R_3$ est Q $CONR_4R_5$.

13. Composés selon la revendication 1 dans lesquels $R_2$ est Q $COOR_6$.

14. Composés selon la revendication 13 dans lesquels $R_1$ est $COOR_7$.

15. Composition herbicide comprenant une quantité herbicide efficace d'un composé selon la revendication 1 et un support.

16. Procédé pour combattre les mauvaises herbes qui comprend leur mise en contact avec une quantité herbicide efficace d'un composé défini dans la revendication 1.

17. Procédé de préparation d'un composé tel que défini dans la revendication 1 qui comprend la réaction d'un composé de formule:

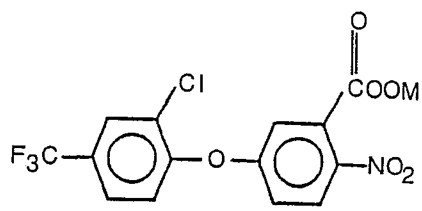

dans laquelle M est tel que défini dans la revendication 1 avec un acide alcanoïque ou un chlorure d'acide alcanoïque de formule

$$X\ CR_1R_2R_3$$

dans laquelle $R_1$, $R_2$ et $R_3$ sont tels que définis dans la revendication 1 et X est un atome d'halogène.

18. Procédé de préparation de composé tel que défini dans la revendication 1 qui comprend la réaction d'un composé de formule:

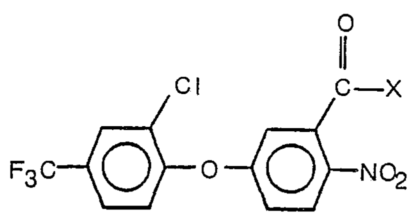

dans laquelle X est halogène, avec un acide alcanoïque de formule

$$HOCR_1R_2R_3$$

dans laquelle $R_1$, $R_2$ et $R_3$ sont tels que définis dans la revendication 1.

**Patentansprüche**

1. Herbizide Verbindungen der Formel

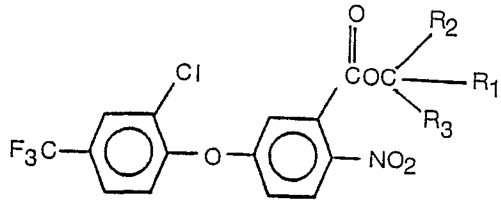

in der

$R_1$ eine der folgenden Bedeutungen hat: COOH; COOM, wobei M ein Alkali-, Erdalkali-, Ammonium-, $Alkyl_{1-5}$ammonium- oder $Dialkyl_{2-6}$ammoniumkation ist; $COOR_7$, wobei $R_7$ $Alkyl_{1-5}$ ist; und

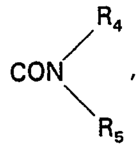 ,

wobei $R_4$ und $R_5$ jeweils Wasserstoff oder $Alkyl_{1-5}$ sind;

$R_2$ und $R_3$ gleich oder verschieden sind und jeweils eine der folgenden Bedeutung haben: Wasserstoff; $Alkyl_{1-5}$; $QCOOR_6$, wobei Q $Alkylen_{0-3}$ und $R_6$ $Alkyl_{1-5}$, Wasserstoff, eine Alkali-, Erdalkali-, Ammonium-, $Alkyl_{1-5}$ammonium- oder $Dialkyl_{2-6}$ammoniumkation ist; oder

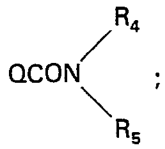 ;

wobei mindestens einer der Substituenten $R_2$ und $R_3$ die Bedeutung

$QCOOR_6$ oder 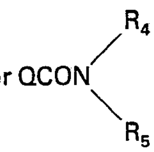

hat.

2. Verbindungen nach Anspruch 1, in denen $R_2$ Wasserstoff ist.

3. Verbindungen nach Anspruch 2, in denen $R_3$ $QCOOR_6$ ist.

4. Verbindungen nach Anspruch 3, in denen $R_1$ $COOR_7$ ist.

5. Verbindungen nach Anspruch 3, in denen Q $Alkylen_{1-3}$ ist.

6. Verbindungen nach Anspruch 3, in denen $R_6$ eines der im Anspruch 1 angegebenen Kationen ist.

7. Verbindungen nach Anspruch 3, in denen $R_6$ Wasserstoff ist.

8. Verbindungen nach Anspruch 3, in denen $R_6$ $Alkyl_{1-5}$ ist.

9. Verbindungen nach Anspruch 3, in denen $R_3$ $COOR_6$ ist.

10. Verbindungen nach Anspruch 3, in denen $R_1$ COOH ist.

11. Verbindungen nach Anspruch 1, in denen $R_2$ $Alkyl_{1-5}$ ist.

12. Verbindungen nach Anspruch 2, in denen $R_3$

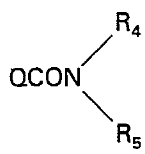

ist.

13. Verbindungen nach Anspruch 1, in denen $R_2$ und $R_3$ (jeweils) $QCOOR_6$ sind.

14. Verbindungen nach Anspruch 13, in denen $R_1$ $COOR_7$ ist.

15. Herbizides Mittel, gekennzeichnet durch eine herbizid wirksame Menge einer Verbindung nach Anspruch 1 und einen Träger dafür.

16. Verfahren zur Bekämpfung unerwünschter Pflanzen, dadurch gekennzeichnet, daß man die Pflanzen mit einer herbizid wirksamen Menge einer Verbindung nach Anspruch 1 in Berührung bringt.

17. Verfahren zur Herstellung einer Verbindung nach Anspruch 1, dadurch gekennzeichnet, daß man eine Verbindung der Formel

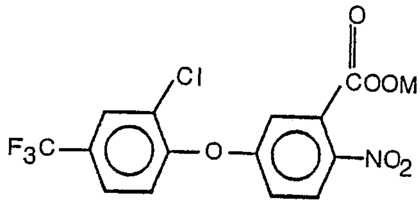

in der M wie in Anspruch 1 definiert ist, mit einer Alkansäure oder einem Halogenid davon der Formel $XCR_1R_2R_3$, in der $R_1$, $R_2$ und $R_3$ wie in Anspruch 1 definiert sind und X ein Halogen ist, zur Umsetzung bringt.

18. Verfahren zur Herstellung einer Verbindung nach Anspruch 1, dadurch gekennzeichnet, daß man eine Verbindung der Formel

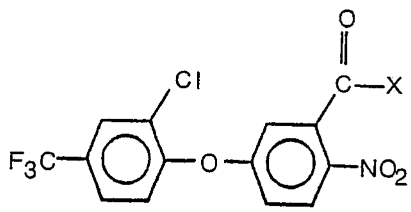

in der X Halogen ist, mit einer Alkansäure der Formel $HOCR_1R_2R_3$, in der $R_1$, $R_2$ und $R_3$ wie in Anspruch 1 definiert sind, zur Umsetzung bringt.